# EUROPEAN PATENT APPLICATION

(11) **EP 4 299 759 A1**
(43) Date of publication of application: **03.01.2024**
(21) Application number: 22382608.2
(22) Date of filing: 29.06.2022
(51) Int. Cl.: C12Q 1/682

(54) **NUCLEIC ACID DETECTION METHOD**

(71) Applicant: Fundación del Sector Público Estatal Centro Nacional de Investigaciones Oncológicas Carlos III (F.S.P. CNIO), 28029 Madrid (ES)
(72) Inventor: CORTÉS LEDESMA, Felipe, 28029 Madrid (ES); GÓMEZ MARÍN, Carlos, 28029 Madrid (ES); MUÑOZ BARRERA, Marta, 28029 Madrid (ES); LÓPEZ DE ALBA, Ernesto, 28029 Madrid (ES)
(74) Representative: Elzaburu S.L.P.

(57) **Abstract**

The present invention relates to a method for detecting the presence of a nucleic acid sequence in a sample using CRISPR technology. The method of the invention allows the detection of nucleic acid amounts as low as 100 fM with a high signal-to-noise ratio and without performing any amplification of the nucleic acid of interest. The present invention also relates to a kit to carry out the method for detecting the presence of a nucleic acid sequence in a sample, and to the use of the method or the kit of the invention for the diagnosis or prognosis of a disease or condition.

## Description

### FIELD OF THE INVENTION

The present invention relates to a method for detecting the presence of a nucleic acid sequence in a sample using CRISPR technology. The method of the invention allows the detection of nucleic acid amounts as low as 100 fM with a high signal-to-noise ratio and without performing any amplification of the nucleic acid of interest. The present invention also relates to a kit to carry out the method for detecting the presence of a nucleic acid sequence in a sample.

### BACKGROUND OF THE INVENTION

The capacity of CRISPR-Cas systems of being programmed to recognize specific nucleic acid sequences has boosted their biotechnological applications. One of them is the detection of the genetic material of pathogens or genetic markers in diagnosis. Systems to for the detection of specific nucleic acid sequences based on CRISPR-Cas technology, namely SHERLOCK, DETECTR and their derivatives, have been recently developed and promise to revolutionize point-of-care diagnostics in the near future. The basis of these systems relies, on the one hand, on the specificity and versatility conferred by their mechanism of target recognition and cleavage, which is directed by base-pair hybridization with a guide RNA (gRNA) sequence that, together with the Cas effector protein, form the Cas ribonucleoprotein (RNP). And, on the other hand, on a robust collateral nuclease activity that some of these Cas proteins acquire when they cleave their target sequence. This collateral activity consists in a sequence-independent unscheduled endonucleolytic degradation, normally of single-stranded nucleic acids of the same type as the sequence-specific target. The detection of this collateral activity by nuclease reporter substrates, either fluorescent or adapted to lateral flow devices, can be therefore used as a readout for the presence of any nucleic acid of interest (genetic material of a pathogen, for example) in a given sample, provided the design of the appropriate Cas RNP. Examples of widely used Cas effector proteins with collateral activity are the Cas13 and Cas12 (including Cas14/Cas12f) families of proteins, activated by and acting on RNA and DNA respectively, although some Cas12a proteins can also degrade dsDNA and ssRNA in addition to ssDNA.

These CRISPR-Cas diagnostic systems have already been proven useful for the detection of a wide range of pathogens and genetic markers. However, despite the great specificity and versatility that these systems offer, they are currently limited by the levels of sensitivity, which are outside the range of the concentrations required for diagnostic purposes and currently rely on pre-amplification of the target sequences. This introduces a complication to the reactions limiting their current use in direct point-of-care applications.

Any improvement in the sensitivity of these assays would therefore have a profound impact towards the widespread application of CRISPR-Cas technology as a reliable diagnostic tool.

Current solutions go in the direction of simplifying the pre-amplification step, for example by isothermal nucleic acid amplification, so reactions can be coupled in the minimal steps and time possible. This, however, does not prevent the requirement of at least some minimal equipment to allow incubation at constant (normally high) temperatures.

The present invention overcomes the problems of the detection methods known up to date and allows the simple, fast and on-site detection and even quantification of nucleic acids present in a sample in very small amounts.

### SUMMARY OF THE INVENTION

A first aspect of the present invention relates to a method for detecting the presence of a nucleic acid sequence in a sample, comprising the following steps:
(a) adding to said sample at least one Cas ribonucleoprotein (Cas RNP) with collateral activity, at least one amplifier, at least one reporter substrate, and, optionally, at least one of an exonuclease or a polymerase, optionally combined with dNTPs and/or NTPs,
(b) incubating the mixture of step (a) for between 1 minute and 8 hours, preferably between 15 minutes and 2 hours, more preferably between 30 minutes and 1.5 hours, at a temperature between 15 to 45 ºC, preferably between 20 to 40 ºC, more preferably between 20 and 37 ºC, and
(c) reading the signal of said reporter substrate;
wherein at least one Cas RNP (target-activatable RNP; T-RNP) is designed to recognize a specific sequence in the nucleic acid of interest (primary target);
wherein the presence of the nucleic acid of interest activates the collateral nucleolytic activity of the T-RNP;
wherein the amplifier is a nucleic acid comprising:
   i) a non-target strand (NTS) that comprises a sequence that is reverse-complementary to the target of a second Cas RNP (amplifier-activatable RNP; A-RNP); and
   ii) a target strand (TS) that comprises a sequence that is, at least in part, reverse-complementary to the NTS;
wherein the 5', 3' or both ends of at least one strand of the amplifier are modified by chemical modification or by circularization;
wherein the collateral activity of activated T- and A-RNPs on the amplifier results in the generation of a TS that is recognizable by and accessible to the A-RNP;
wherein the reporter substrate comprises the type of nucleic acid that is target for the collateral activity of the A-RNP, optionally wherein its ends are protected against degradation or polymerization; and
wherein no amplification of the nucleic acid sequence of interest is performed.

A second aspect of the present invention relates to a kit for the detection of the presence or absence or for the quantification of a nucleic acid of interest in a sample, comprising at least one Cas RNP with collateral activity, at least one amplifier, at least one reporter substrate and, optionally, at least one of an exonuclease or a polymerase, optionally combined with dNTPs and/or NTPs,
wherein at least one Cas RNP (target-activatable RNP; T-RNP) is designed to recognize a specific sequence in the nucleic acid of interest (primary target);
wherein the amplifier is a nucleic acid comprising:
   i) a non-target strand (NTS) that comprises a sequence that is reverse-complementary to the target of a second Cas RNP (amplifier-activatable RNP; A-RNP); and
   ii) a target strand (TS) that comprises a sequence that is, at least in part, reverse-complementary to the NTS;
wherein the 5', 3' or both ends of at least one strand of the amplifier are modified by chemical modification or by circularization;
wherein the reporter substrate comprises the type of nucleic acid that is target for the collateral activity of the A-RNP, optionally wherein its ends are protected against degradation or polymerization; and
wherein the kit does not comprise means for amplifying the nucleic acid sequence of interest in the sample.

A third aspect of the present invention relates to the use of the method of the first aspect or the kit of the second aspect, for the detection of the presence or absence or for the quantification of a nucleic acid of interest in a sample, preferably wherein said nucleic acid is present in the sample in a concentration of between 100 fM and 10 nM.

A fourth aspect of the present invention relates to the use of the method of the first aspect or the kit of the second aspect, for the diagnosis or prognosis of a disease or condition, wherein the presence or absence or amount of the nucleic acid is associated to said disease or condition.

### DESCRIPTION OF THE INVENTION

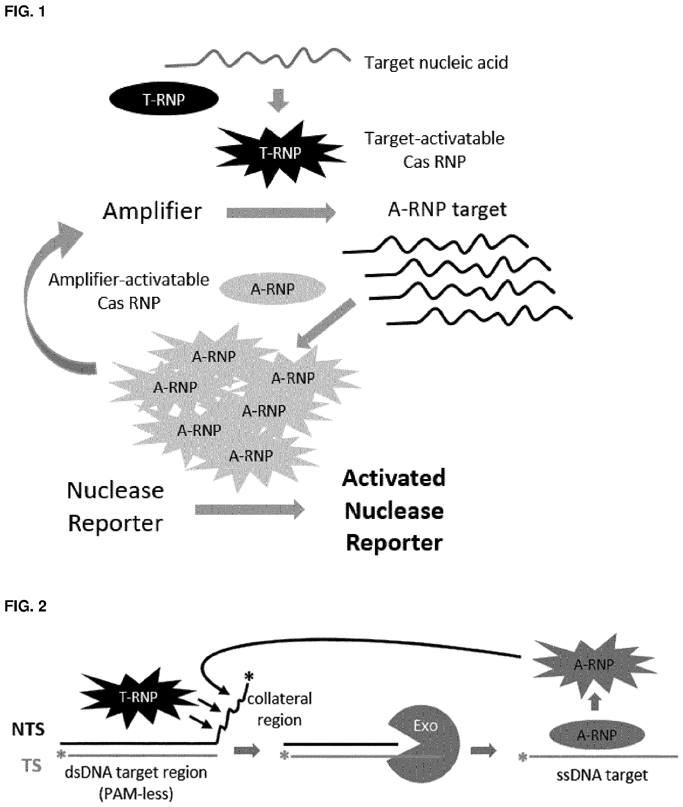

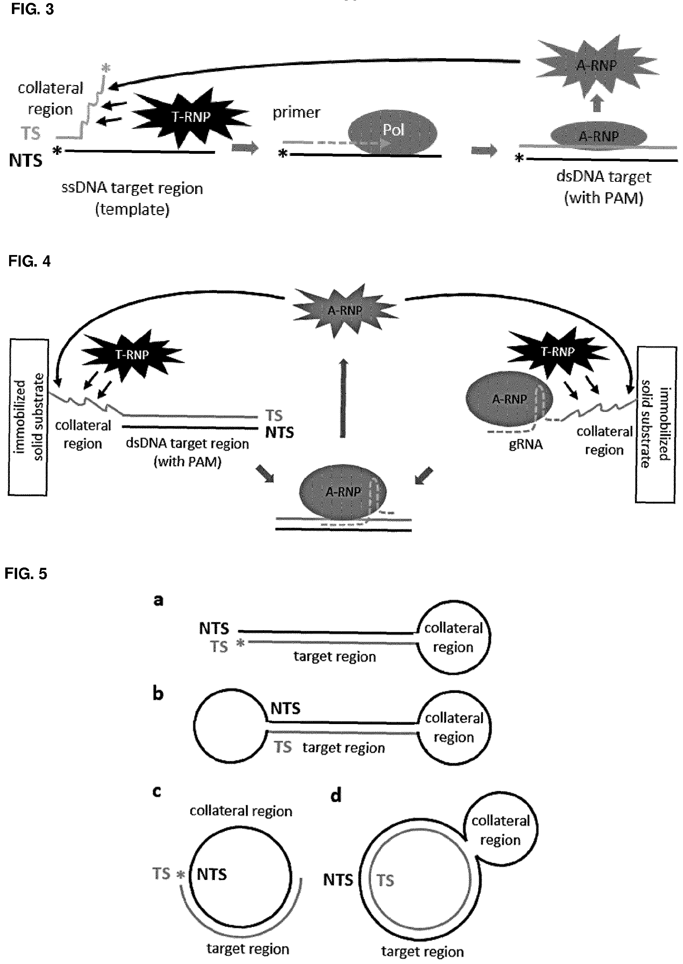

The basis of the present invention is a chain reaction of effector Cas protein activity by which its collateral *in trans* nucleolytic activity on a specific nucleic acid molecule, the amplifier, leads, alone or coupled to other enzymatic activities, to the release or appearance of more target molecules, unleashing a self-primed chain reaction that eventually reaches full activation of reporter substrates, even with minimal initial concentrations of the target of interest (Figure 1). Thus, the activation of a first target-inducible Cas T-RNP by the nucleic acid sequence of interest leads to the activation of amplifier molecules that activate a second amplifier-inducible Cas A-RNP, which also activates additional amplifier molecules, triggering the self-fueled Cas activity cascade. For this, the amplifiers contain a target region that is initially inert for A-RNP recognition but, upon the degradation of a specific region in its structure (collateral region) by Cas RNP collateral activity, a recognizable target for the A-RNP is released and/or reconstituted. This is achieved by either having a concealed target that is exposed by the action of an exonuclease (Figure 2), by an absent target that is synthesized by a polymerase (Figure 3) or by a spatial separation between amplifier target region and A-RNP (Figure 4). In the context of this CRISPR-Cas Chain Reaction (3CR or CCR), and in analogy to PCR, one can use end-point measurements for qualitative detection of a nucleic acid of interest, or real time reactions for quantitative analysis.

Direct detection of specific nucleic acid sequences with CRISPR-Cas systems at concentrations normally found in biological or clinical samples is currently limiting and require methods, such as PCR, that amplify the target of interest. This imposes the need of more complex reactions and advanced equipment, limiting the capacity of point-of-care testing. As explained before, the present invention bypasses this need by dramatically increasing the sensitivity of direct CRISPR-Cas nucleic acid detection. In contrast to currently used methods, the present invention does not amplify the nucleic acid sequence of interest, but instead amplifies Cas protein activation, simply boosting the signal without the need of additional pre-amplification steps. It is thus faster and easier, occurring in a single, or at least sequential reaction, without the need of further liquid handling and at a wide range of temperatures that are compatible with point-of-care testing.

The term "T-RNP" or "Target-activatable RNP", as used herein, refers to the Cas RNP that is activated by the presence of the target sequence, i.e. of the nucleic acid of interest that is detected by the method of the present invention. The T-RNP comprises a gRNA that hybridizes with the nucleic acid to be detected by the method of the present invention. The term "A-RNP" or "Amplifier-activatable RNP", as used herein, refers to the Cas RNP that is activated by the presence of the target sequence in the in the TS of the amplifier molecule. Thus, the A-RNP comprises a gRNA that hybridizes with the target sequence in the TS of the amplifier. The term "activated" as used herein refers to the CasRNPs, that are activated when their gRNA hybridizes with their target sequence and said target sequence is cleaved. The activated CasRNPs that are used in the present invention show collateral activity.

In step (a) of the method of the first aspect, the Cas RNPs can be added already preassembled or also as individual components, namely a Cas protein and a guide RNA (gRNA); the rest of components can be added and incubated together, or some may be pre-incubated before adding the rest in different sub-steps. In step (a), a buffer suitable for CasRNP enzymatic reactions, as well as for exonuclease and/or polymerase reactions, is used. A buffer may be, for example, buffer 2.1 NEB (New England Biolabs), which comprises at 1X concentration 50 mM NaCl, 10 mM TrisHCl, 10 mM MgCl₂, 100 µg/ml BSA.

In a preferred embodiment of the first aspect, the nucleic acid to be detected is a single stranded or double stranded nucleic acid. In a preferred embodiment of the first aspect, the nucleic acid to be detected is RNA or DNA. In another preferred embodiment of the method of the first aspect, the nucleic acid of interest is RNA. In another preferred embodiment of the method of the first aspect, the nucleic acid of interest is DNA. In a preferred embodiment, the nucleic acid of interest is from SARS-CoV2. This preferred embodiment applies for the method of the first aspect, the kit of the second aspect and the use of the third aspect.

The method of the first aspect, the kit of the second aspect and the use of the third aspect have all as preferred embodiment those where the nucleic acid of interest is from a virus, a bacterium, a mammal, a human or is a genetic trait, a mutation, a disease marker, such as a cancer marker, etc., including single nucleotide polymorphisms.

In a preferred embodiment of the first aspect, the exonuclease and/or polymerase is Exonuclease I, Exonuclease III, Exonuclease T, Rec JF, T7 exonuclease, Lambda exonuclease, T4 DNA polymerase, T7 DNA polymerase, DNA polymerase I, Klenow fragment, Phi29 DNA polymerase.

In a preferred embodiment of the first aspect, a phosphatase is added in step (a), preferably a polynucleotide kinase-phosphatase, more preferably T4 polynucleotide kinase-phosphatase, preferably when a Cas13 RNP is used.

In a preferred embodiment of the method of the first aspect, the TS comprises fully, partially or does not comprise the target region of the A-RNP.

In a preferred embodiment of the method of the first aspect, the amplifier comprises one or more nucleic acid molecules, preferably no more than three molecules in total, and more preferably comprises two molecules: one single molecule for each the NTS and the TS.

In a preferred embodiment of the method of the first aspect, 3', 5' or both ends of at least one of the molecules of the amplifier are chemically modified to prevent degradation by exonucleases and/or priming of nucleic acid synthesis by polymerases. In another preferred embodiment at least one of the nucleic acid molecules is ligated to another DNA strand or circularized by ligation between its 5' and 3' ends (Figure 5).

In a preferred embodiment of the method of the first aspect, the amplifier comprises a collateral region comprised by at least one extension in the TS and/or NTS, preferably the collateral region is single-stranded DNA (ssDNA) and/or RNA (ssRNA); and wherein, optionally, this collateral region comprises at least one strand (TS or NTS) that is protected from the collateral activity of the Cas RNPs.

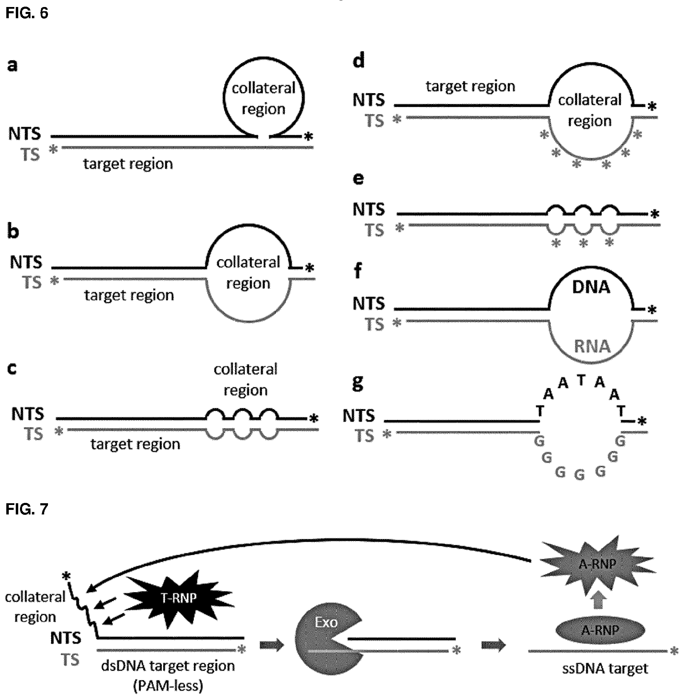

Regardless of the structure (Figures 2-6), this collateral region is the preferential target for the collateral activity of the Cas RNPs. The presence of this so-called collateral region increases the efficiency of the method of the invention, since the collateral activity is directed towards a region where it is productive for the release and/or synthesis of the TS, for example by comprising a nucleic acid type that is the preferred target of the collateral activity of the chosen Cas RNP (e.g. RNA *vs.* DNA, ssDNA vs. dsDNA, etc) (Figures 2-4 and 6f). The collateral activity may be additionally prevented from acting on particular strands and/or regions where it is not productive for example by including chemical modifications, such as phosphorothioate linkages, or specific nucleotide sequences that are resistant (such as the polyG for Cas12a) (Figures 6d-g).

In another preferred embodiment of the method of the first aspect, at least one exonuclease is added in step (a), and the amplifier comprises a complete target region in the TS but does not comprise a PAM motif, and the collateral region is located on the NTS, either 3' (Figure 2) or 5' of the target region (Figure 7), coinciding with the polarity of the exonuclease used.

As used herein, the term "PAM motif" refers to the protospacer adjacent motif (PAM), which is a 2-6-base pair DNA sequence immediately following the DNA sequence targeted by the Cas RNP. The presence of this PAM motif near the target sequence is necessary for dsDNA Cas RNPs to bind and cleave the molecule in the target sequence. The PAM motif is not relevant for ssRNA-directed Cas proteins such as Cas13.

The term "exonuclease" refers to the enzymatic activity, it also includes polymerases with exonuclease activity, such as that of T4 and T7 DNA polymerases.

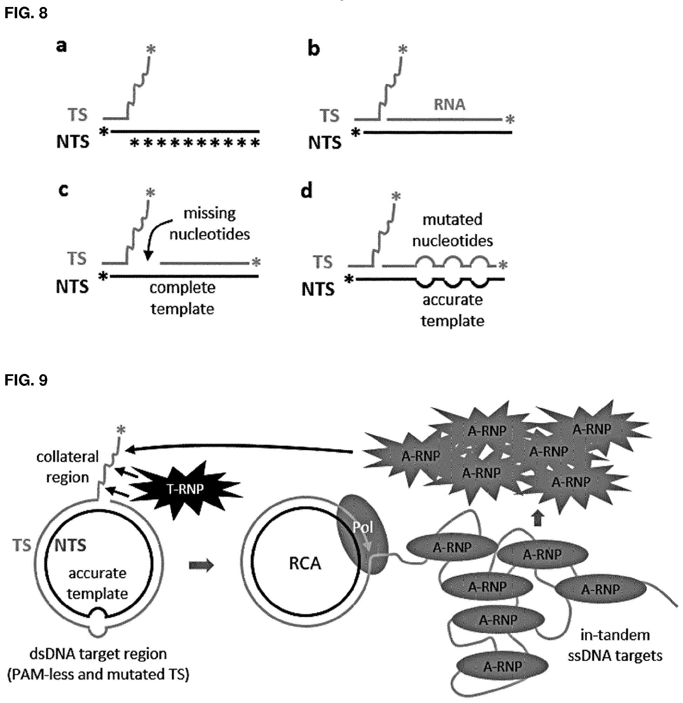

In another preferred embodiment of the method of the first aspect, at least one polymerase and dNTPs (deoxynucleotide triphosphates) or NTPs (nucleotide triphosphates) are added in step (a) and wherein the TS does not fully comprise the target region or comprises at least one mutated or missing nucleotide, or is at least partially comprised by a nucleic acid type not recognized by the A-RNP; and wherein the collateral region is located on the TS, 5' of these modifications (Figures 3 and 8).

In a preferred embodiment of the method of the first aspect (Figure 4), the amplifier contains a PAM motif and a complete target region in the TS, and is attached to a solid substrate through the collateral region(s), wherein the gRNA of the A-RNP also contains at least one collateral region through which it is attached to a different solid substrate; and wherein the two solid substrates are spatially separated;

In a preferred embodiment of the method of the first aspect, a single Cas RNP is used, and the target region of the amplifier NTS comprises a sequence that is reverse-complementary to the primary target sequence in the nucleic acid of interest; the T-RNP therefore acts also as A-RNP.

In another preferred embodiment of the method of the first aspect, the first and second Cas RNP comprise the same Cas protein, but a different gRNA, or a different Cas protein.

In a preferred embodiment, at least one Cas RNP comprises a Cas12 protein, preferably Cas12a, more preferably *Lachnospiraceae bacterium* Lba Cas12a. In another preferred embodiment, at least one Cas RNP comprises a Cas13 protein, preferably Cas13a, more preferably *Leptotrichia wadei* Lwa Cas13a.

In a preferred embodiment, both T-RNP and A-RNP comprise a Cas12 protein, preferably Cas12a, more preferably Lba Cas12a.

In a preferred embodiment, both T-RNP and A-RNP comprise a Cas13 protein, preferably Cas13a, more preferably Lwa Cas13a.

In a preferred embodiment, the T-RNP comprises a Cas13 protein, preferably Cas13a, more preferably Lwa Cas13a, and the A-RNP comprises a Cas12 protein, preferably Cas12a, more preferably Lba Cas12a.

In another preferred embodiment of the method of the first aspect, the amplifier is prepared by mixing the TS and NTS in a ratio between 1:1 and 1:5, preferably between 1:1 and 1:3, more preferably between 1:1 and 1:2.

In another preferred embodiment of the method of the first aspect, the non-annealed TS and/or NTS is removed from the reaction or quenched with the reverse-complementary strand; in another preferred embodiment, correctly assembled amplifiers are purified by PAGE or HPLC before adding the amplifier in step (a).

In another preferred embodiment of the method of the first aspect, the amplifier is a dsDNA lacking a PAM motif, comprising the target sequence of a Cas12 A-RNP, and wherein the NTS has a 3' extension of ssDNA and/or RNA terminally protected from exonucleolytic degradation; and wherein a 3' exonuclease is added in step (a), optionally wherein a phosphatase is also added in step (a).

In a preferred embodiment, the reporter substrate is a nucleic acid of between 2 and 100 nucleotides. In a preferred embodiment, the reporter substrate is between 3 and 10, more preferably between 4 and 8, even more preferably between 4 and 6 or 5 nucleotides long. In a preferred embodiment, the reporter substrate contains markers, which can be for example a fluorophore/quencher pair attached to each end of the nucleic acid, or a molecule such as biotin or fluorescein, etc, that can be further detected by streptavidin or specific antibodies. For fluorescent detection, the reporter contains a fluorophore/quencher pair attached to each end of the nucleic acid. The nucleic acid is short (typically 5 nucleotides) to allow quenching of the fluorescence unless it is degraded, but of sufficient length to allow the action of the activated Cas RNPs. The structure of the reporter is known by the skilled person and can be designed using the general knowledge in the field. Fluorescence is typically measured using a fluorimeter. For quantitative analysis, a standard curve generated with known concentrations of the nucleic acid is used. In an analogous manner to quantitative PCR, for quantitative assays, the fluorescence is measured and the time when half of the maximum fluorescence signal is detected, is used for the quantification. For example, for lateral flow detection, the reporter contains two motifs that can be specifically recognized, one at each end of the nucleic acid. One is used for capture/immobilization and the other one for detection, so cleaved and uncleaved molecules can be distinguished. Preferably biotin is used for immobilization and fluorescein is used for detection with specific antibodies. Since the length of the molecule is less relevant in this case, the amplifier itself can be used as a reporter by adding the biotin and fluorescein moieties to the strand that is subject to degradation. Therefore, in a preferred embodiment, the amplifier is also the reporter substrate.

The term "56-FAM" or "FAM", as used herein, refers to the marker fluorescein. The term "3IABkFQ", as used herein, refers to the quencher Iowa Black^{®} FQ, which has a broad absorbance spectrum ranging from 420 to 620 nm with peak absorbance at 531 nm. This quencher is ideal for use with fluorescein.

In another preferred embodiment of the method of the first aspect, the reporter is a nucleic acid molecule with a fluorescent marker in one end and a quencher in the other end.

In another preferred embodiment of the method of the first aspect, steps (a) and (b) are performed in sub-steps:
(a1) adding to said sample at least one T-RNP with collateral activity and at least one amplifier,
(b1) mixing and incubating the mixture of step (a1) between 1 minute and 2 hours, preferably between 10 minutes and 1 hours, more preferably between 20 minutes and 40 minutes, at a temperature between 15 to 45 ºC, preferably between 20 to 40 ºC, more preferably between 25 and 37 ºC,
(a2) optionally adding more amplifier and/or at least one of its components (TS and NTS), preferably adding additional NTS of the amplifier,
(b2) optionally mixing and incubating the mixture of step (a2) between 1 minute and 1 hour, preferably between 10 minutes and 1 hour, more preferably between 15 minutes and 30 minutes, at a temperature between 15 to 45 ºC, preferably between 20 to 40 ºC, more preferably between 25 and 37 ºC,
(a3) adding at least one A-RNP, at least one reporter substrate, and at least one of an exonuclease or a polymerase, optionally combined with dNTPs and/or NTPs, and (b3) incubating the mixture of step (a3) between 1 minute and 8 hours, preferably between 15 minutes and 2 hours, more preferably between 30 minutes and 1.5 hours, at a temperature between 15 to 45 ºC, preferably between 20 to 40 ºC, more preferably between 25 and 37 ºC.

In another preferred embodiment, sub-steps (a2) and (b2) are omitted.

In a preferred embodiment of the first aspect, the T-RNP comprises a Cas13 protein, and the amplifier comprises a DNA TS containing a target region for a Cas12 A-RNP and with at least one phosphorothioate linkage, and a DNA NTS without PAM motif, containing at least one phosphorothioate linkage, and comprising a single-stranded collateral region with at least 3 ribonucleotides.

In another preferred embodiment of the method of the first aspect, the T-RNP comprises a Lwa Cas13a protein and a gRNA of SEQ ID NO: 6, the A-RNP comprises a Lba Cas12a protein and a gRNA of SEQ ID NO: 3, and the amplifier comprises SEQ ID NO:4 and SEQ ID NO: 5 as TS and NTS, respectively.

In a preferred embodiment of the first aspect (Figure 9), the A-RNP comprises a Cas12 protein, and the amplifier comprises a circularized NTS lacking a PAM motif and a TS fully or partially hybridized to the NTS but harbouring at least one mutation in the target region and a non-annealed DNA and/or RNA flap with at least one phosphorothioate linkage and an InvdT motif on its 3' end, and a DNA polymerase with strand-displacement capacity and 3' exonuclease activity, preferably phi29pol is added in step (a).

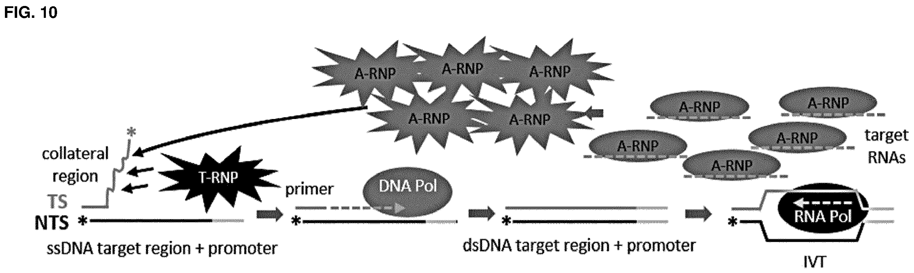

In a preferred embodiment of the first aspect (Figure 10), both the T- and A-RNPs comprise a Cas13 protein; the amplifier comprises a NTS with at least one phosphorothioate linkage and an InvdT motif on its 3' end, and comprising the non-transcribed strand for the target of the Cas13 A-RNP and a promoter sequence for a RNA polymerase, preferably T7 RNA polymerase, and a TS partially hybridized to the 3' end of the NTS with a flap containing at least 3 ribonucleotides, at least one phosphorothioate linkage and an InvdT motif at its 3' end. DNA and RNA polymerases, preferably T7 RNA polymerase, are added in step (a).

In another preferred embodiment, the method of the first aspect of the present invention is used in a diagnostic method (*in vitro* method): said diagnostic method comprises the method for the detection of the presence of a nucleic acid sequence in a sample of the first aspect, and also comprises associating the presence or absence, or the amount of the nucleic acid sequence in the sample to a disease or condition. The method can be performed in a qualitative or quantitative manner, for example, using a lateral-flow device, the method can be performed for qualitative detection, while by measuring the fluorescence of the reporter in real time using a fluorometer, the method can be quantitative. As explained above, the time when the level of fluorescence reaches half of the maximum fluorescence is used for quantification. Particular embodiments of interest are as shown in the examples, a SARS-CoV2 quantitative detection method or a SARS-CoV2 qualitative detection method which uses a lateral-flow device.

In relation to the second aspect of the present invention, the kit can be a kit-of-parts and also a device, such as a lateral-flow device. Thus, the term "kit", as used herein, means both a kit-of-parts and a device.

In relation to the kit of the second aspect of the invention, the means for amplifying the nucleic acid sequence of interest in the sample are, for example, a thermoresistant DNA polymerase for PCR, a reverse polymerase that copies cDNA from RNA or specific primer pairs for amplifying a sequence by PCR or other amplification means, such as Loop-mediated isothermal amplification (LAMP) or Recombinase polymerase amplification (RPA).

In a preferred embodiment, the kit comprises a T-RNP that comprises a Cas13a protein, preferably Lwa Cas13a protein, and a gRNA of SEQ ID NO: 6, a A-RNP that comprises a Cas12a protein, preferably Lba Cas12a, and a gRNA of SEQ ID NO: 3, and an amplifier that comprises SEQ ID NO:4 and SEQ ID NO: 5 as TS and NTS, respectively. This kit is useful for detecting the presence and also quantifying the amount of nucleic acid from SARS-CoV2.

In a preferred embodiment of the second aspect, the kit further comprises the components required for lateral-flow detection, or is a lateral-flow device, as described in Gootenberg et al. 2018 (Science, 360(6387):439-444) and Broughton et al. 2020 (Nature Biotechnology, 38(7):870-874), and wherein the kit optionally comprises means for taking a sample and/or for extracting nucleic acids from a sample.

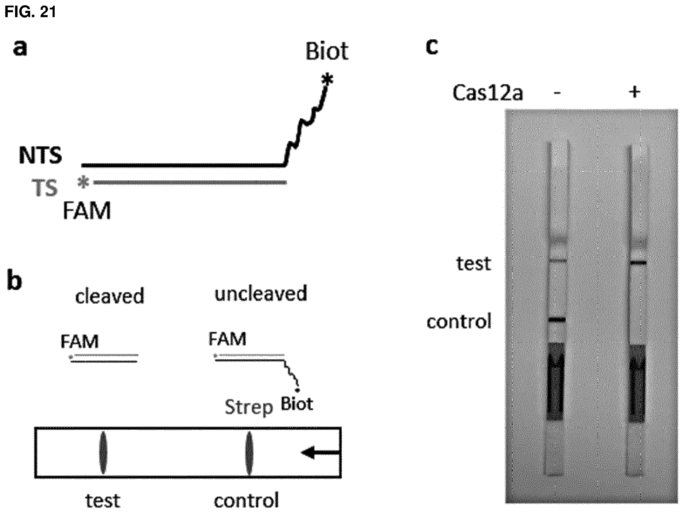

In a lateral flow kit, the sample is applied on one end (pad) of a solid support, through which it migrates by capillarity. The lateral-flow reporter substrate is a nucleic acid molecule with two different marker molecules at each end, preferably biotin and fluorescein. The solid support contains two regions with immobilized molecules that bind each of these makers, and through which the sample migrates sequentially, preferably first through a stripe coated with streptavidin proximal to the pad, and preferably then through a stripe coated with anti-fluorescein antibodies distal to the pad. One of the markers, preferably fluorescein, is used for detection of the reporter, preferably with anti-fluorescein antibodies conjugated to gold nanoparticles that are embedded in the pad, while the other marker, preferably biotin, is used for retention at the first stripe, preferably by interaction with streptavidin. When the reporter reaches the first stripe, all the uncleaved molecules will be retained by interaction with the retention marker, while the fragments of cleaved reporter containing the detection marker will continue migrating through the solid support until reaching the second stripe. Visualization of signal only in the first stripe (control region) is indicative of the absence of the nucleic acid of interest in the sample, while visualization of signal in the second stripe (test region) is indicative of the presence of the nucleic acid of interest in the sample. This type of lateral flow test is shown in Figure 21.

As used herein, the expression "at least one" means one, two, three or more, preferably means one or two, more preferably means one.

In a preferred embodiment of the first and second aspect, the amplifier is modified to directly act as reporter for lateral flow by including retention and visualization markers (as described above) at either end of the collateral region, either on the same strand or one marker on each strand.

The method, kit and use of the present invention can be adapted to the detection of proteins or metabolites of interest by attaching nucleic acids to specific antibodies or binding molecules, and then detecting these nucleic acids. Thus, this is a preferred embodiment for the four aspects of the present invention.

All the above embodiments of the first aspect also apply to the second, third and fourth aspects. All the embodiments of the second aspect apply also to the first, third and fourth aspects. All the embodiments of the third aspect apply also the first, second and fourth aspects. All the embodiments of the fourth aspect apply also to the first, second and third aspects. The present disclosure includes all the combinations of all the aspects disclosed above. The term "comprising" or "comprise" or "comprises" includes the term "consisting of" or "consist of" or "consists of".

### BRIEF DESCRIPTION OF FIGURES

**Figure 1****:** General scheme of CRISPR Cas Chain Reaction (3CR). The target activates a first Cas RNP (Target-activatable RNP or T-RNP). Each activated T-RNP acts on amplifier molecules releasing or generating targets for a second amplifier-activatable Cas RNP (A-RNP), which becomes activated. Activated A-RNPs act on the amplifier unleashing a chain reaction of target release and RNP activation. Amplified Cas activity is detected with reporter substrates (fluorescence, lateral flow device, etc).
**Figure 2****:** Scheme of an example of target-exposure 3CR approach with a 3' exonuclease activity. The amplifier contains a target for a Cas12a A-RNP in dsDNA form (target region), but the lack of a PAM motif prevents recognition. The non-target strand (NTS) has a 3' extension of ssDNA or RNA (collateral region) terminally protected from exonucleolytic degradation (asterisk). Activated T-RNP degrades the overhang generating the substrate for a 3' exonuclease that degrades the NTS and releases the target strand (TS) is a ssDNA form. The now accessible TS activates A-RNPs, which can further remove overhangs from more amplifiers, unleashing the chain reaction.
**Figure 3****:** Scheme of a target-synthesis 3CR approach. The amplifier contains the non-target strand (NTS) for a Cas12a A-RNP that acts as template, and a partially hybridized target strand (TS) nucleic acid with a complementary region and a non-complementary flap of ssDNA/RNA on the 3' end (collateral region) terminally protected from 3' exonucleolytic degradation (asterisk). Activated T-RNP degrades the flap, generating a primer that the DNA polymerase can extend synthesizing the full dsDNA A-RNP target. Note that the target region needs to contain a PAM motif for A-RNP recognition in a dsDNA form. Reconstituted target activates A-RNP, which then further removes the flap from more amplifiers, unleashing the chain reaction.
**Figure 4****:** Scheme of a spatial separation approach. An amplifier with a fully functional target region is attached through the collateral region to a solid substrate and spatially separated from the A-RNP, which is attached to a different solid substrate through a collateral region in its gRNA. Activated T-RNP cleaves the collateral regions releasing both the target region of the amplifier and the A-RNP, so they can now interact leading to the activation of the latter, and whose collateral activity liberates more molecules unleashing 3CR amplification.
**Figure 5****:** Examples of amplifier configurations in which DNA ends are protected by circularization. NTS and TS are connected in hairpin-loop (a) or dumbbell (b) structures. Alternatively, either the NTS alone (c), or both the NTS and TS (d) are circularized.
**Figure 6****:** Examples of different collateral-region configurations (left) and strategies to direct Cas activity (right). Loops (a), bubbles (b) and arrayed mismatches (c) are used as collateral region. If necessary, collateral RNP activity is directed to the NTS by protecting the NTS with chemical modifications (d, e), or using a different nucleic acid type (f) or sequence (g).
**Figure 7****:** Scheme of an example of target-exposure 3CR approach with a 5' exonuclease activity. As in Figure 1, but with a 5' extension on the NTS and a 5' exonuclease activity.
**Figure 8****:** Examples of different strategies to protect the NTS template from degradation by activated RNPs. The NTS is protected by chemical modifications (a) and/or by hybridization with RNA (b) or with DNA, in which case missing (c) or mutated (d) nucleotides are included to prevent direct A-RNP activation.
**Figure 9****:** Scheme of a rolling-circle amplification (RCA) 3CR approach. The amplifier contains a circular non-target strand (NTS) for a Cas12a A-RNP, but lacking a PAM motif, that is annealed to a target strand (TS) with mutations that further prevent direct recognition by the A-RNP. The TS is not circularized and contains a non-complementary flap of ssDNA/RNA on the 3' end (collateral region) terminally protected from 3' exonucleolytic degradation (asterisk). Activated T-RNP degrades the flap generating a substrate for a DNA polymerase with strand displacement capacity to trigger a RCA reaction, which synthesizes and liberates in-tandem repeats of a non-mutated TS in a ssDNA form. Thus, each event activates multiple A-RNPs, which further removes the flap from more amplifiers, unleashing a more potent chain reaction.

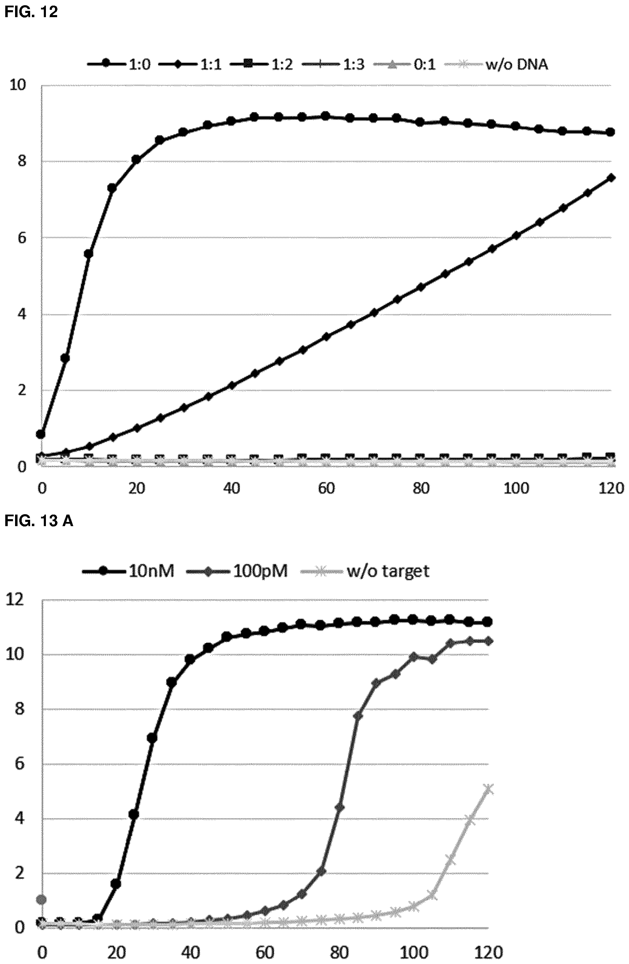

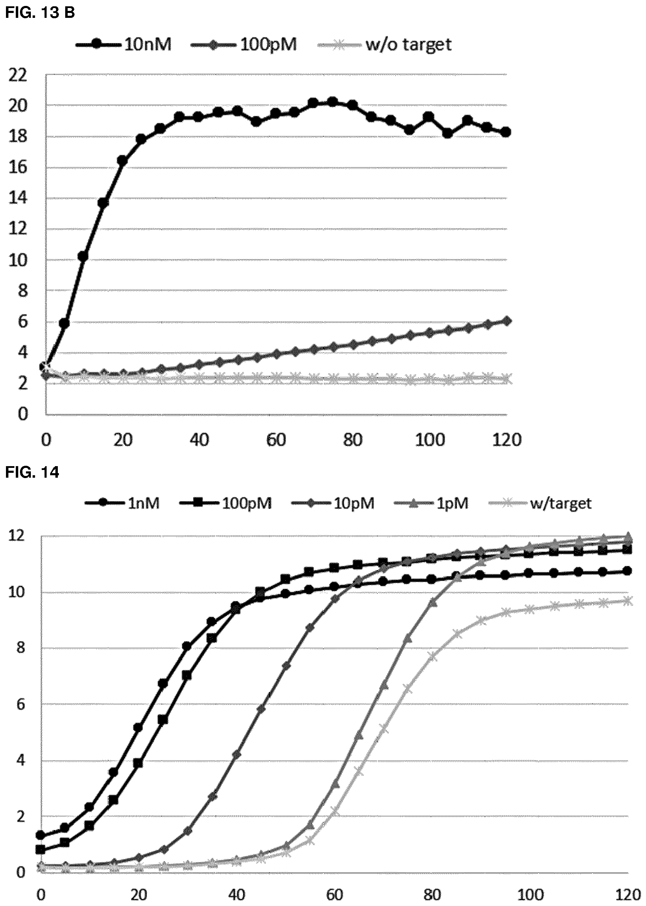

**Figure 10****:** Scheme of an *in vitro* transcription (IVT) 3CR approach. The approach is based on target synthesis, as in Figure 13, but with the following modifications: The TS and NTS correspond to transcribed and non-transcribed strands, respectively, as, when reconstituted in a dsDNA form by the DNA polymerase, the target region is transcribed by the RNA polymerase, generating RNA molecules that are target for the Cas13a A-RNP. For this, a promoter sequence is included in the NTS. Thus, each event activates multiple A-RNPs, which can then further remove the flap from more amplifiers, unleashing a more potent chain reaction.

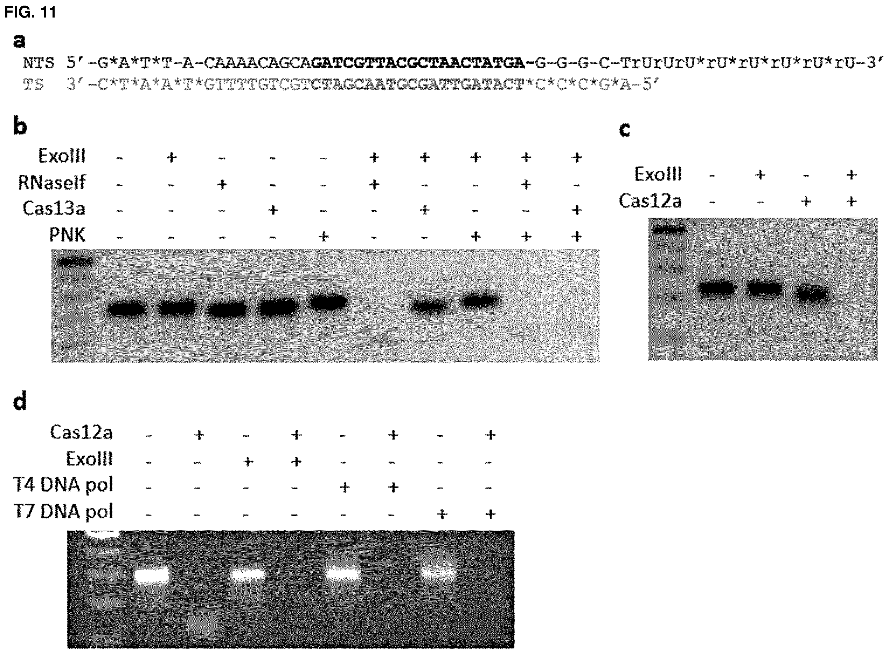

**Figure 11****:** Design and validation of a target exposure 3' exonuclease amplifier. (a) Scheme of the amplifier molecule with SEQ ID NO: 4 as TS and SEQ ID NO: 5 as NTS. Ribonucleotides are indicated with (r). The target region is highlighted in bold. Protective phosphorothioate linkages are indicated with asterisks. This amplifier was incubated for 1h at 37°C in NEB Buffer 2.1 with (b) LwaCas13a and (c,d) LbaCas12a, in the presence (+) or absence (-) of the indicated enzymes, and run on a 3% agarose gel and stained with Etidium Bromide.
**Figure 12****:** Background activation of A-RNPs by amplifier molecules. The amplifiers described in Figure 3 were prepared by annealing the indicated ratios of TS:NTS, and incubated at 37ºC for the indicated times in the presence of the corresponding LbuCas12a A-RNP and a fluorescent DNAse reporter substrate. Fluorescence was measured at 5 min intervals for 2 hours. The abscissa axis indicates time in minutes and the ordinate axis indicates fluorescence in arbitrary units.
**Figure 13****:** Example 1: One-step target-exposure 3CR DNA detection at room temperature and comparison with direct detection. 3CR (a) and direct detection (b) reactions were performed at 25ºC in the presence of the indicated concentrations of target DNA as described in Example 1. Fluorescence emission of a DNAse reporter substrate was measured at 5 min intervals for 2 hours. The abscissa axis indicates time in minutes and the ordinate axis indicates fluorescence in arbitrary units.
**Figure 14****:** Example 2: Two-step target-exposure 3CR DNA detection with Exolll. 3CR reactions were performed as described in Example 2 in the presence of the indicated concentrations of target DNA. Fluorescence emission of a DNAse reporter substrate was measured at 5 min intervals for 2 hours. The abscissa axis indicates time in minutes and the ordinate axis indicates fluorescence in arbitrary units.

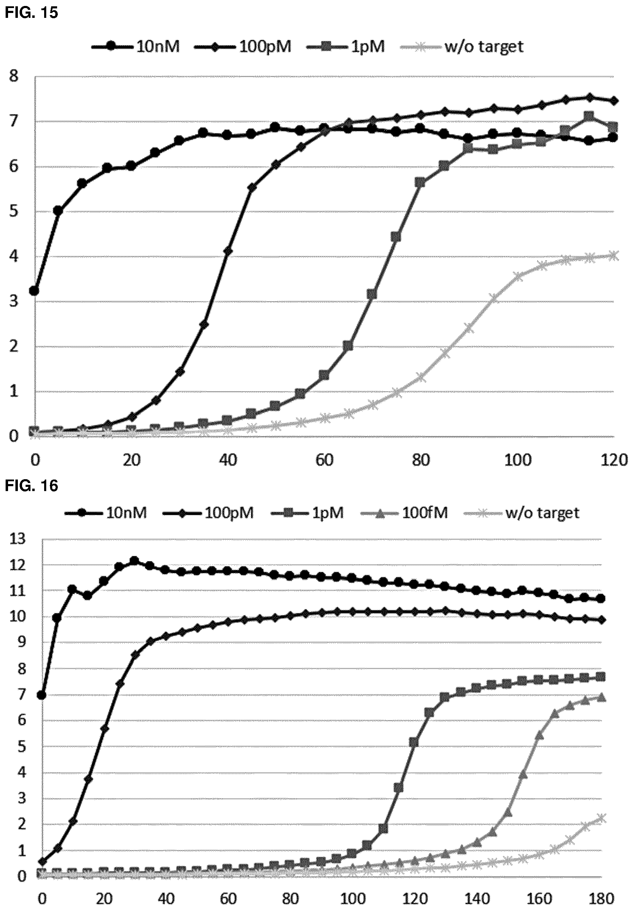

**Figure 15****:** Example 3: Two-step target-exposure 3CR DNA detection with T7 pol. 3CR reactions were performed as described in Example 3 in the presence of the indicated concentrations of target DNA. Fluorescence emission of a DNAse reporter substrate was measured at 5 min intervals for 2 hours. The abscissa axis indicates time in minutes and the ordinate axis indicates fluorescence in arbitrary units.
**Figure 16****:** Example 4: Thee-step target-exposure 3CR DNA detection with T4 pol. 3CR reactions were performed as described in Example 4 in the presence of the indicated concentrations of target DNA. Fluorescence emission of a DNAse reporter substrate was measured at 5 min intervals for 3 hours. The abscissa axis indicates time in minutes and the ordinate axis indicates fluorescence in arbitrary units.

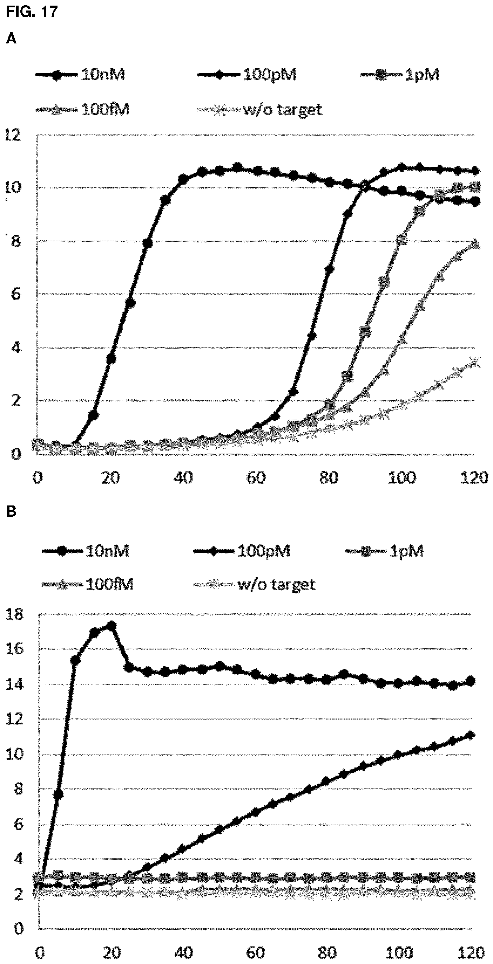

**Figure 17****:** Example 5: Two-step target-exposure 3CR RNA detection with Exolll and comparison with direct detection. 3CR (a) and direct detection (b) reactions were performed as described in Example 5 in the presence of the indicated concentrations of target RNA. Fluorescence emission of a DNAse (a) or RNAse (b) reporter substrate was measured at 5 min intervals for 2 hours. The abscissa axis indicates time in minutes and the ordinate axis indicates fluorescence in arbitrary units.

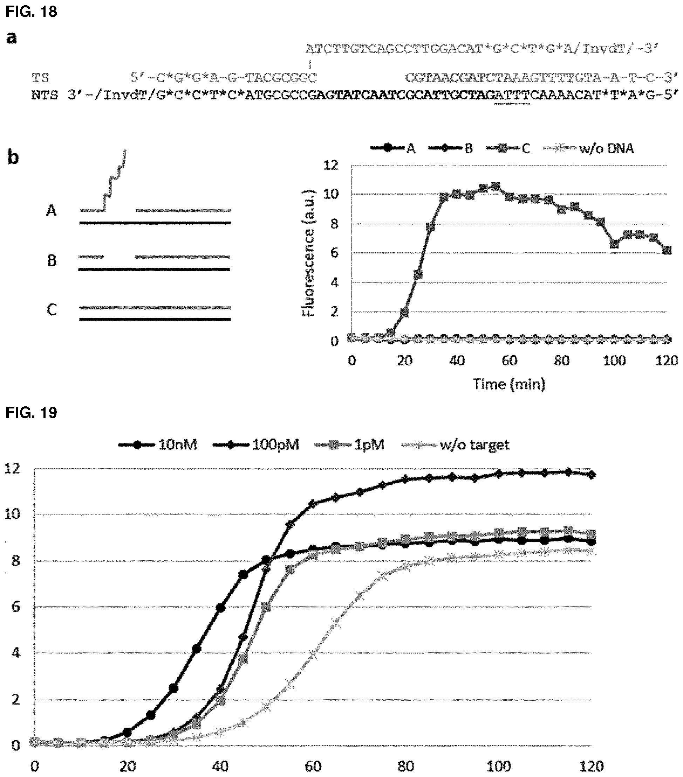

**Figure 18****:** Design and validation of a target-synthesis amplifier. (a) Scheme of the amplifier comprising SEQ ID NO: 8, SEQ ID NO: 9 and SEQ ID NO: 10. PAM motif is underlined, and target region is highlighted in bold. Asterisks indicate phosphorothioate linkages and 3' ends modified with an inverted dT nucleotide are indicated (/InvdT/). For the validation of the strategy, we demonstrated that only a fully dsDNA reconstituted amplifier elicited a Cas12a response (b) and that Cas12a activity was required for Klenow (Kle) to reconstitute full dsDNA sequence of the amplifier.
**Figure 19****:** Example 6: Target synthesis-3CR DNA detection with Klenow. 3CR reactions were performed as described in Example 6 in the presence of the indicated concentrations of target RNA. Fluorescence emission of a DNAse reporter substrate was measured at 5 min intervals for 1.5 hours. The abscissa axis indicates time in minutes and the ordinate axis indicates fluorescence in arbitrary units.

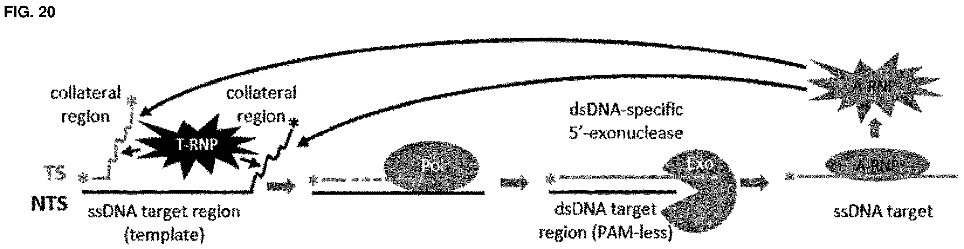

**Figure 20****:** Scheme of a double-hit 3CR approach. A combination of the target synthesis (Figure 13) and 5' target exposure (Figure 7) approaches, imposes the need of two events to achieve productive 3CR amplification, limiting potential background signal due to spontaneous hydrolysis of collateral regions. Upon activity of the T-RNP on the collateral region of the target strand (TS), and subsequent polymerization from the primer generated, a dsDNA target region is reconstituted, but the lack of PAM motif prevents activation of the Cas12a A-RNP, unless the NTS is degraded by a dsDNA-specific 5' exonuclease, which requires degradation of the NTS collateral region by the T-RNP. Once the reaction is triggered, it proceeds through the action of the activated A-RNPs as in other 3CR approaches. The NTS is protected from the action of activated RNPs as described in Figure 14.
**Figure 21****:** Example of dual amplifier-reporter molecules for lateral-flow detection. By adding biotin and FAM molecules to the 3' ends of NTS and TS, as depicted in (a), the amplifier also acted as a reporter for lateral-flow detection. (b) The biotin was retained in the control region by a streptavidin-coated region, limiting the mobility of anti-FAM gold nanoparticles if the amplifier was intact. When it was cleaved and/or degraded, however, the nanoparticles continued to the test region. (c) Treatment of these dual amplifier-reporter molecules with activated LbuCas12a was detected as a complete shift towards the test position.

### EXAMPLES

The following examples illustrate the present invention and are not to be understood as limiting the invention. In the examples, nucleic acid sequences related to SARS-CoV-2 are detected, but the claimed technology described herein can easily be adapted by the skilled person to any nucleic acid of interest, including other viruses, bacteria, genetic traits, cancer markers, etc., including the identification of single nucleotide polymorphisms.

In the simplest embodiment, the method of the invention is based on the use of a Cas protein, such as those of the Cas12 family, that only recognize their target sequence in a doublestranded form (dsDNA for Cas12 proteins) if they contain an adjacent PAM motif, while this is not necessary to detect its target strand when single-stranded. The same applies to Cas proteins, such as the Cas13 family, that only recognize single-stranded nucleic acids (RNA in this case) and do not have a PAM motif. With this in mind, as can be seen in Figure 2, we envisioned amplifier molecules containing a dsDNA region (target region) comprised by both target and non-target stands (TS and NTS respectively) for a Cas12a RNP (amplifier-activatable Cas12a RNP; A-RNP). The non-target strand contains on its 3' end a non-annealed ssDNA region which is a preferential substrate for collateral degradation by activated Cas12a (collateral region). Both the TS and NTS are protected from degradation by a 3' exonuclease that will be present in the reaction by chemical modification and/or binding to bulky adducts. Although not essential, 5' ends can be also protected in the same way to prevent degradation by other exonucleases that may be present in the sample. Thus, only in the presence of a given target sequence of interest, the endonucleolytic collateral activity of a target-activated Cas12a RNP (T-RNP) acts on the collateral region of the NTS and releases a free 3' nucleic acid end that can be used by the 3' exonuclease to release the TS in a ssDNA form recognizable by the A-RNP, which will in turn act on the collateral region of additional amplifiers and so on, unleashing exponential 3CR signal amplification.

This target-exposure approach is also useful to detect RNA by using, for example, a Cas13abased T-RNP; in which case the collateral region of the amplifier contains ribonucleotides to allow collateral cleavage by activated Cas13a. With this in mind, we generated an amplifier molecule based on a previously described DNA target of LbaCas12a with a mutated PAM, adding on the NTS a 3' extension of 8 uracil ribonucleotides, and with the last 5 containing phosphorothioate linkages (Figure 11a) (see oligonucleotide list in Example 1), and checked the action of LwaCas13a, LbaCas12a and the 3' exonuclease *E. coli* Exo III on this substrate. As can be seen in Figure 11b, the amplifier was fully protected from the action of Exo III. Incubation with active LwaCas13a or RNAse If (as a positive control), however, resulted in cleavage of the overhang, and a partial degradation of the molecule with the release of target strand if Exo III was included in the reaction. Furthermore, as expected, the addition of T4 polynucleotide kinase-phosphatase (PNK) strongly stimulated the reactions. This was because the phosphatase activity of the enzyme removed 2'-3' cyclophosphate products of Cas13a collateral activity that limited subsequent 3' exonucleolytic activity. Interestingly, incubation with active LbaCas12a also resulted in cleavage of the overhang (Figure 11c), due to the capacity of its collateral activity to act on either ssDNA, dsDNA or ssRNA. Further incubation with Exo III resulted, as expected, in full degradation of the amplifier, since the Cas12a collateral activity also degraded released ssDNA TS. These results demonstrate the validity of amplifiers based on RNA overhangs to amplify both Cas13a and Cas12a activity, and therefore, to specifically detect both RNA and DNA sequences. Amplifiers with both ssDNA and ssRNA, or a combination of ssDNA- and ssRNA-based amplifiers can also be used. We also obtained similar results for Cas12a activation with two other enzymes such as T4 and T7 DNA polymerases (Figure 11d), which, in the absence of dNTPs have strong 3' exonuclease activities on both ssDNA and dsDNA.

All amplifier TS molecules should be annealed to the corresponding NTS, as any free TS present could be sufficient to trigger the reaction. In order to ensure that this is the case, we tested the capacity of amplifier molecules annealed with different TS:NTS ratios to activate its corresponding Cas12a A-RNP. As shown in Figure 12, TS alone (1:0 ratio) elicited a robust activation of the A-RNP as measured by the time-dependent emission of fluorescence by the single-stranded DNAse reporter substrate, while, as expected, the NTS alone (0:1 ratio) did not result in measurable A-RNP activation. Furthermore, the presence of the NTS in an equimolar 1:1 ratio significantly reduced TS-dependent A-RNP activation but was not completely eliminated, unless an excess was used (1:2 ratio and above). From this we conclude that free unannealed TS is a source of background signal that can be removed by preparing the amplifiers with a 1:2 ratio TS:NTS.

Although an overhang is the simplest configuration for the collateral region, additional structures, such as for example loops, bubbles or serial mismatches, can be used (Figure 5a-c). In this case, if required, different approaches can be adopted to direct the collateral activity towards the strand of the collateral region in which it is productive for the purpose of TS release. For example, in bubble structures the TS within the collateral region can be protected by chemical modification (e.g. phosphorothioate) or by nucleic acid types or sequences that are disfavored substrates for the collateral activity (e.g. RNA and poly-G regions for Cas12a) (Figure 5d-g). Related with the structure of the amplifier, it is worth noting that another possibility for protecting DNA ends, besides chemical modification, is the circularization of the given stands, which can be achieved in different configurations as shown in Figure 4. Furthermore, it is worth noting that, for DNA detection, a single Cas12a RNP can be used by basing the amplifier on the sequence of interest, so that the same Cas12a RNP recognizes both the primary target and the released TS from the amplifier. Finally, if a 5' instead of a 3' exonuclease is used; in which case the collateral region of the amplifier is located on the 5' end of the NTS (Figure 6).

### Example 1: One-step detection of a ssDNA sequence related to SARS-CoV2 with LbaCas12a and E. coli exonuclease III (Exo III).

We used the amplifier described above (Figure 11) and an LbaCas12a T-RNP directed against an already validated ssDNA region derived from SARS-CoV2 *gene S.* For the 3CR reactions, different concentrations of a synthetic ssDNA of the target region were mixed with freshly prepared amplifier in a 1:2 ratio TS:NTS, LbaCas12a T-RNP, LbaCas12a A-RNPs, Exo III, PNK and a fluorescent DNAse reporter protected from exonucleolytic degradation (see oligonucleotide list for details), and then incubated for 2 hours at 25ºC in a Victor Nivo device (Perkin Elmer), taking measurements of reporter fluorescence every 5 min. The results in Figure 13a show that a 100 pM concentration of target can be unambiguously detected in less than 90 min. In contrast, detection of 100 pM target in the absence of amplifier in these room temperature conditions only results in weak reporter fluorescence (Figure 13b). It is worth noting that, in addition to the increase in sensitivity, the self-activating nature of 3CR allows achieving full reporter fluorescence regardless of target concentration, which, similarly to real-time PCR, simply determines the time required to achieve exponential amplification. This characteristic not only allows quantitative measurements using real-time detection, as proven by this example, but is also important to adapt the methodology to qualitative analysis in low-sensitivity detection devices such as lateral flow systems. This demonstrates the potential of 3CR as an amplification-free method for room-temperature detection of nucleic acids.

### Oligonucleotide list:

| Name | Sequence |
|---|---|
| LbaCas12a gRNA against S gene of SARS-CoV2 | UrArArUrUrUrCrUrArCrUrArArGrUrGrUrArGrArUrArUrArGrArArAr ArGrUrCrCrUrArGrGrUrUrGrA (SEQ ID NO: 1) |
| Sequence of nucleic acid of interest (S gene of SARS-CoV2) | CGGCCGTCGTTTTACATTTTTTCAACCTAGGACTTTTCTATTA AAATATAATTTTTCAGGAAACAGCTATGAATT (SEQ ID NO: 2) |
| LbaCas12a gRNA against amplifier target | rUrArArUrUrUrCrUrArCrUrArArGrUrGrUrArGrArUrGrArUrCrGrUr UrArCrGrCrUrArArCrUrArUrGrA (SEQ ID NO: 3) |
| Target Strand (TS) for target exposure | A*G*C*C*C*TCATAGTTAGCGTAACGATCTGCTGTTTTG*T*A*A *T*C (SEQ ID NO: 4) |
| Non-Target Strand (NTS) for 3' target exposure | G*A*T*TACAAAACAGCAGATCGTTACGCTAACTATGAGGGCTr UrUrU*rU*rU*rU*rU*rU (SEQ ID NO: 5) |
| Cas12a reporter | /56-FAM/T*TAT*T*/3IABkFQ/ |

| | |
|---|---|
| * Indicates phosphorothioate linkages in nucleotides or ribonucleotides marked | |

### Reaction conditions:

| | |
|---|---|
| LbaCas12a T-RNP | 50 nM |
| LbaCas12a A-RNP | 50 nM |
| Cas12a reporter | 200 nM |
| Amplifier | 50 nM |
| ExoIII (NEB) | 0.05 U/ul (1U) |
| T4 PNK (NEB) | 0.125 U/ul (2.5U) |
| Buffer 2.1 NEB 1X | 50 mM NaCl |
| | 10 mM TrisHCl |
| | 10 mM MgCl₂ |
| | 100 µg/ml BSA |
| RNAase Inhibitor, Murine (NEB) | 1 U/ul (20 U) |

| | |
|---|---|
| Temperature: 25ºC. Time: 120 min with measurements every 5 min. | |

### Example 2: Two-step detection of a ssDNA sequence related to SARS-CoV2 with LbaCas12a and E. coli exonuclease III (Exo III).

The main problem for the sensitivity of 3CR as determined in Example 1 is the background signal that starts to appear around 100 min even in the absence of target DNA (Figure 13a). This likely results from an intrinsic capacity of the amplifier to activate the A-RNP, even in dsDNA form, and/or by residual action of Exo III on the amplifiers so that it releases a minimal amount of TS for A-RNP activation. This problem was minimized to some extent by preincubating the target with T-RNP and amplifier, and then adding the rest of the components, which were responsible for this background signal. As can be seen in Figure 14, this preincubation delayed the appearance of unspecific signal, allowing us to achieve full reporter activation with only 10 pM target in 60 min.

### Oligonucleotide list:

As in Example 1.

### Reaction conditions:

| | |
|---|---|
| LbaCas12a T-RNP | 50 nM |
| Amplifier | 50 nM |
| T4 PNK (NEB) | 0.125 U/ul (2.5U) |
| Buffer 2.1 NEB 1X | As described above |
| RNAase Inhibitor, Murine (NEB) | 1 U/ul (20 U) |

| | |
|---|---|
| Temperature: 37ºC. Time: 45 minutes. | |

Add:

| | |
|---|---|
| LbaCas12a A-RNP | 50 nM |
| Cas12a reporter | 200 nM |
| Exo III (NEB) | 0.05 U/ul (1U) |
| Buffer 2.1 NEB 1X | As described above |

| | |
|---|---|
| Temperature: 25ºC. Time: 120 min with measurements every 5 min. | |

### Example 3: Two-step detection of a ssDNA sequence related to SARS-CoV2 with LbaCas12a and the 3' exonucleolytic activity of T7 DNA polymerase (T7 pol).

We tested other 3' exonuclease activities other than Exo III, and turned our attention to T7 pol, in line with the results of Figure 11d. We set up 3CR reactions, essentially as described in Example 2, but with T7 pol instead of Exolll. As can be seen in Figure 15, we achieved full detection of 1 pM target in around 80 min incubation, with only minimal background at this time.

### Oligonucleotide list:

As in Example 1.

### Reaction conditions:

| | |
|---|---|
| LbaCas12a T-RNP | 50 nM |
| Amplifier | 50 nM |
| T4 PNK (NEB) | 0.125 U/ul (2.5U) |
| Buffer 2.1 NEB 1X | As described above |
| RNAase Inhibitor, Murine (NEB) | 1 U/ul (20 U) |

| | |
|---|---|
| Time: 45 minutes. Temperature: 37ºC. | |

Add:

| | |
|---|---|
| LbaCas12a A-RNP | 50 nM |
| Cas12a reporter | 200 nM |
| T7 pol (NEB) | 0.005 U/ul (0.1U) |
| Buffer 2.1 NEB 1X | As described above |

| | |
|---|---|
| Temperature: 37ºC. Time: 120 min with measurements every 5 min. | |

### Example 4: Three-step detection of a ssDNA sequence related to SARS-CoV2 with LbaCas12a and the 3' exonucleolytic activity of T4 DNA polymerase (T4 pol).

We also tested T4 pol. We included a preincubation step with amplifiers generated with a 1:1 ratio TS:NTS, then added an excess of NTS to bring it to the 1:2 ratio, and finally incubated with the rest of the components. As can be seen in Figure 16, this three-step reaction further increased the sensitivity of the assay, reaching the detection of 100 fM in 160 min, before background signal starts to appear.

### Oligonucleotide list:

As in Example 1.

### Reaction conditions:

| | |
|---|---|
| LbaCas12a T-RNP | 50 nM |
| Amplifier | 50 nM |
| T4 PNK (NEB) | 0.125 U/ul (2.5U) |
| Buffer 2.1 NEB 1X | As described above |
| RNAase Inhibitor, Murine (NEB) | 1 U/ul (20 U) |

| | |
|---|---|
| Time: 45 minutes. Temperature: 37ºC. | |

Add:

| | |
|---|---|
| Non-target Strand 8rU | 50 nM |

| | |
|---|---|
| Time: 30 minutes. Temperature: 37ºC. | |

Add:

| | |
|---|---|
| LbaCas12a A-RNP | 50 nM |
| Cas12a reporter | 200 nM |
| T4 pol (NEB) | 0.015 U/ul (0.3U) |
| Buffer 2.1 NEB 1X | As described above |

| | |
|---|---|
| Temperature: 37ºC. Time: 180 min with measurements every 5 min. | |

### Example 5: Two-step detection of SARS-CoV2-related ssRNA sequences with LwaCas13a, LbaCas12a and E. coli exonuclease III (Exo III).

We used the amplifiers and 2-step incubation approach described in Example 2 to extend the capabilities of 3CR into RNA detection. For this, we used LwaCas13a as T-RNP, which was specifically directed against a target sequence in SARS-CoV2 *gene S.* We also used Exolll for the proven activity extending ribonucleolytic cleavage (Figure 11b). The results in Figure 17a show that we were able to detect 100 fM of an *in vitro*-transcribed RNA target in 120 min, in contrast to the lower than 1 pM sensitivity of the detection in the absence of the amplifier (Figure 17b). This demonstrates to what an extent 3CR can also potentiate RNA detection.

### Oligonucleotide list:

| Name | Sequence |
|---|---|
| LwaCas13a gRNA against *S gene* of SARS-CoV2 | rGrArUrUrUrArGrArCrUrArCrCrCrCrArArArArArCrGrArArGrGrGr GrArCrUrArArArArCrGrCrArGrCrArCrCrArGrCrUrGrUrCrCrArAr CrCrUrGrArArGrArArG (SEQ ID NO: 6) |
| IVT DNA template for SARS-CoV *S gene* (T7 promoter is underlined) | rGrArUrUrUrArGrArCrUrArCrCrCrArArArArCrGrArArGrGrGr GrArCrUrArArArArCrGrCrArGrCrArCrCrArGrCrUrGrUrCrCrArAr CrCrUrGrArArGrArArG (SEQ ID NO: 6) TAATACGACTCACTATAGGTAACATCACTAGGTTTCAAACTT TACTTGCTTTACATAGAAGTTATTTGACTCCTGGTGATTCTT CTTCAGGTTGGACAGCTGGTGCTGCAGCTTATTATGTGGGT TATCTTCAACCTAGGACTTTTCTATTAAAATATAATGAAAATG GAACCATTACAGATGCTGTAGACTGTGC (SEQ ID NO: 7) rUrArArUrUrUrCrUrArCrUrArArGrUrGrUrArGrArUrGrArUrCrGrUr UrArCrGrCrUrArArCrUrArUrGrA (SEQ ID NO: 3) A*G*C*C*C*TCATAGTTAGCGTAACGATCTGCTGTTTTG*T*A* A*T*C (SEQ ID NO: 4) G*A*T*TACAAAACAGCAGATCGTTACGCTAACTATGAGGGC TrUrUrU*rU*rU*rU*rU*rU (SEQ ID NO: 5) |
| LbaCas12a gRNA against amplifier target | rUrArArUrUrUrCrUrArCrUrArArGrUrGrUrArGrArUrGrArUrCrGrUr UrArCrGrCrUrArArCrUrArUrGrA (SEQ ID NO: 3) |
| Target Strand (TS) for target exposure | A*G*C*C*C*TCATAGTTAGCGTAACGATCTGCTGTTTTG*T*A* A*T*C (SEQ ID NO: 4) |
| Non-Target Strand (NTS) for 3' target exposure | G*A*T*TACAAAACAGCAGATCGTTACGCTAACTATGAGGGC TrUrUrU*rU*rU*rU*rU*rU (SEQ ID NO: 5) |
| Cas12a reporter | /56-FAM/T*TAT*T*/3IABkFQ/ |

### Reaction conditions:

| | |
|---|---|
| LwaCas13a T-RNP | 50 nM |
| T4 PNK | 0.125 U/ul (2.5U) |
| Buffer 2.1 NEB 1X | As described above |
| RNAase Inhibitor, Murine (NEB) | 1 U/ul (20 U) |
| Amplifier | 50 nM |

| | |
|---|---|
| Time: 45 minutes. Temperature: 37ºC. | |

Add:

| | |
|---|---|
| LbaCas12a A-RNP | 50 nM |
| Buffer 2.1 NEB 1X | As described above |
| Cas12a reporter | 200 nM |
| Exonuclease III | 0.05 U/ul (1U) |
| Temperature: 25ºC. Time: 120 min with measurements every 5 min. | |

We therefore demonstrated that 3CR is a useful method for detecting specific sequences of both DNA and RNA without target amplification, at room temperature and with a sensitivity that can go up to 100 fM.

As discussed above, an excess of NTS is advantageous to limit direct activation by the amplifier. Another advantageous possibility is to prepare the amplifier with a 1:2 TS:NTS ratio as described in the examples, and then quench the excess of NTS with a non-activating TS (for example containing mutations in the target sequence). Other advantageous embodiments include a step for purifying correctly assembled amplifiers by PAGE or HPLC.

The absence of gRNA hybridization with the 4 last PAM-distal nucleotides of the target, strongly affects Cas12a cleavage and activation with dsDNA, even in the presence of a PAM motif, while minimally affecting in a ssDNA form. Another advantageous embodiment is achieved by using this or other reduced targets by either changing the sequence in the amplifier or the gRNA used in the A-RNP. Systematic and/or massive screening methods are available to the skilled person to identify the optimal target sequences for this purpose. In another embodiment, other Cas proteins, either natural or engineered, with a stronger preference for PAM-less ssDNA than Cas12a, are used.

In another embodiment, the target sequence is not originally present in the amplifier, and it is then synthesized, instead of exposed, in the course of the reaction. In the simplest configuration (Figure 3), the TS does not cover the target region but only its adjacent upstream region, like a primer annealed to the 3' end of the NTS. This TS primer contains an unannealed flap in its 3' end, conforming the collateral region of the amplifier, so that it prevents extension from the primer unless it is previously degraded. All 3' ends in the amplifier are blocked from exonuclease activity, as described above, and contain additional modifications to block priming (inverted dT, spacer, biotin-streptavidin, circularization, etc,). Collateral Cas activity together with proofreading 3' exonuclease activity of a DNA polymerase results in full degradation of the flap so the annealed 3' terminus can be used for complete synthesis of the TS using the NTS as template and reconstitution of a dsDNA target molecule for the A-RNP, triggering thus the 3CR reaction. Please note that being dsDNA, and in contrast to the target-exposure approach, this amplifier sequence contains a PAM motif. In addition, the ssDNA region in the target strand is protected from degradation by collateral activity of the activated RNPs (Figure 7). This is achieved by including protective modifications such as phosphorothioates or by hybridizing to DNA or RNA, in which case key nucleotides in the TS are missing or mutated to prevent direct activation of the A-RNP. Please note that the different adaptations and structures described above for target-exposure are also applied to these target-synthesis approaches, including detection of both DNA and RNA, different end-protection possibilities and collateral region configurations, in which case, a particular strand in the collateral region is selectively protected (as in Figure 5d-g but protecting the NTS).

### Example 6: Target-synthesis 3CR DNA detection with LbaCas12a and the Klenow fragment of E. coli DNA polymerase I (Klenow).

For the target-synthesis approach we used the same target sequence in the amplifier as in Example 1, and designed a structure formed by the NTS and a primer containing a 25-nucleotide ssDNA flap annealed to its 3' end (Figure 18a). A protective DNA oligonucleotide was also annealed to the 5' target region of the NTS but leaving a 10-nucleotide ssDNA gap, which was sufficient to prevent A-RNP activation (Figure 18b). All 3' ends in the amplifier, except for the protective oligonucleotide, were protected with phosphorothioate linkages and contained an inverted dT residue to prevent their use as primers. Please note that an excess of NTS was not required during the preparation of this type of amplifiers. When checked by agarose gel electrophoresis, the amplifier molecules were not affected by incubation with Klenow alone, but a full conversion into a dsDNA form was observed when activated LbaCas12a was included in the reaction.

We used this amplifier molecule and performed 3CR reactions, essentially as described in Example 1, but with Klenow instead of T4 pol/PNK, and the addition of dNTPs for DNA synthesis. No preincubation was included, so reactions were directly placed at 37ºC during 2h, with continuous analysis of reporter activation by fluorescence emission. As can be observed in Figure 19 1 pM target could be detected in 50 min of incubation in these conditions.

### Oligonucleotide list:

| Name | Sequence |
|---|---|
| LbaCas12a gRNA against S gene of SARS-CoV2 | rUrArArUrUrUrCrUrArCrUrArArGrUrGrUrArGrArUrArUrArGrArAr ArArGrUrCrCrUrArGrGrUrUrGrA (SEQ ID NO: 1) |
| Sequence of nucleic acid of interest (S gene of SARS-CoV2) | CGGCCGTCGTTTTACATTTTTTCAACCTAGGACTTTTCTATT AAAATATAATTTTTCAGGAAACAGCTATGAATT (SEQ ID NO: 2) |
| LbaCas12a gRNA against amplifier target | rUrA rArUrU rUrCrU rArCrU rArArG rUrGrU rArGrA rUrGrA rUrCrG rUrUrA rCrGrC rUrArA rCrUrA rUrGrA (SEQ ID NO: 3) |
| Target Strand (TS) Primer for target-synthesis | C*G*G*AGTACGCGGCATCTTGTCAGCCTTGGACAT*G*C*T* G*A/InvdT/ (SEQ ID NO: 8) |
| Non-Target Strand (NTS) for target synthesis | G*A*T*TACAAAACTTTAGATCGTTACGCTAACTATGAGCCGC GTA*C*T*C*C*G/InvdT/ (SEQ ID NO: 9) |
| Target Strand (TS) Cover for target synthesis | CGTAACGATCTAAAGTTTTGTAATC (SEQ ID NO: 10) |
| Cas12a reporter | /56-FAM/T*TAT*T*/3IABkFQ/ |

| | |
|---|---|
| InvdT means inverted deoxythymidine 3'- monophosphate. | |

### Reaction conditions:

| | |
|---|---|
| LbaCas12a T-RNP | 50 nM |
| Amplifier | 50 nM |
| LbaCas12a A-RNP | 50 nM |
| Cas12a reporter | 200 nM |
| dNTPs | 250 uM |
| Klenow | 0.25 U/ul (5U) |
| Buffer 2.1 NEB 1X | As described above |
| RNAase Inhibitor, Murine (NEB) | 1 U/ul (20 U) |

Figure 8 shows an approach based on the spatial separation of amplifier and A-RNP. The gRNA of the A-RNP contains a collateral region with a terminal biotin moiety, or any modification that allows its binding to a solid substrate. The amplifier is also bound to a different solid substrate through similar means. The reaction is carried out with a spatial separation of both solid substrates, and hence of the amplifier and the A-RNP. RNP collateral activity releases both the target region of the amplifier and the A-RNP, allowing activation of the latter and triggering the 3CR reaction. An advantageous possibility in terms of specificity is to use molecules with more than one collateral region-dependent attachment to the solid substrate, and/or combining this spatial-separation approach with target exposure or synthesis by modifying the amplifier and including enzymatic activities in the reaction as described above. Figure 9 shows an approach that uses a circularized NTS and an annealed TS that covers the entire circle and a flap that constitutes the collateral region in the 3' end. The target region does not contain a PAM motif and the TS harbours mutations, to strongly prevent direct recognition by the A-RNP. The advantage of this embodiment is that, once polymerization is triggered by flap degradation, DNA synthesis can proceed through rolling-circle amplification (RCA), provided that a DNA polymerase with strand displacement capacity (such as for example phi29pol) is used, releasing multiple in-tandem copies of the TS, without mutations and in a ssDNA form, so that they are perfectly recognizable by the A-RNPs. With this system each activated amplifier results in the generation of multiple targets (one after each "lap" of RCA synthesis), truly boosting signal amplification.

Figure 10 shows an approach that includes the reconstitution of a transcription unit for an RNA polymerase (such as, for example, T7 RNA polymerase), whose transcription generates a target RNA molecule that activates a Cas13-based A-RNP. This presents two advantages. First, as in the RCA-based example above, each activated amplifier leads to the generation of multiple amplifier targets. Second, if applied to RNA detection, there are no DNAses present in the reaction, so the NTS does not need to be protected from degradation.

Figure 20 shows an approach for reducing the background where the amplifier requires two independent nucleolytic events combining subsequent target synthesis and exposure systems. 3CR can be adapted to qualitative diagnosis with lateral flow devices. For this, the amplifier itself is used as a reporter, simply by adding biotin and FAM moieties to the ends of the NTS, or one in the TS and the other one in the NTS (Figure 21a), so that only uncleaved molecules are retained in the control strip (Figure 21b). Such an amplifier allowed the detection of Lba Cas12a DNAse activity by a lateral flow strip (Figure 21c), and bypassed the need to use a specific reporter molecule in this setting.

## Claims

1. A method for detecting the presence of a nucleic acid sequence in a sample, comprising the following steps:
(a) adding to said sample at least one Cas ribonucleoprotein (Cas RNP) with collateral activity, at least one amplifier, at least one reporter substrate, and, optionally, at least one of an exonuclease or a polymerase, optionally combined with dNTPs and/or NTPs,
(b) incubating the mixture of step (a) for between 1 minute and 8 hours, preferably between 15 minutes and 2 hours, more preferably between 30 minutes and 1.5 hours, at a temperature between 15 to 45 ºC, preferably between 20 to 40 ºC, more preferably between 20 and 37 ºC, and
(c) reading the signal of said reporter substrate;
wherein at least one Cas RNP (target-activatable RNP; T-RNP) is designed to recognize a specific sequence in the nucleic acid of interest;
wherein the presence of the nucleic acid of interest activates the collateral nucleolytic activity of the T-RNP;
wherein the amplifier is a nucleic acid comprising:
i) a non-target strand (NTS) that comprises a sequence that is reverse-complementary to the target of a second Cas RNP (amplifier-activatable RNP; A-RNP); and
ii) a target strand (TS) that comprises a sequence that is, at least in part, reverse-complementary to the NTS;
wherein the 5', 3' or both ends of at least one strand of the amplifier are modified by chemical modification or by circularization;
wherein the collateral activity of activated T- and A-RNPs on the amplifier, results in the generation of a TS that is recognizable by and accessible to the A-RNP;
wherein the reporter substrate comprises the type of nucleic acid that is target for the collateral activity of the A-RNP, optionally wherein its ends are protected against degradation or polymerization; and
wherein no amplification of the nucleic acid sequence of interest is performed.

2. The method of claim 1, wherein the amplifier comprises a collateral region comprising at least one extension in the TS and/or NTS, preferably the collateral region comprises ssDNA and/or ssRNA; and optionally wherein said collateral region comprises at least one strand in the TS or NTS that is protected from the collateral activity of the T-RNP or A-RNP.

3. The method of claim 2, wherein at least one exonuclease is added in step (a), and wherein the amplifier comprises a complete target region in the TS but does not comprise a PAM motif and the collateral region is located on the NTS, either 3' or 5' of the target region, coinciding with the polarity of the exonuclease used.

4. The method of claim 2 wherein at least one polymerase and dNTPs or NTPs are added in step (a) and wherein the TS does not fully comprise the target region or comprises at least one mutated or missing nucleotide; and wherein the collateral region is located on the TS, 5' of these modifications.

5. The method of any one of claims 1 to 4, wherein the T-RNP and A-RNP have the same guide RNA and/or the same Cas protein.

6. The method of any one of claims 1 to 5, wherein the nucleic acid of interest is RNA.

7. The method of any one of claims 1 to 6, wherein the amplifier is prepared by mixing the TS and NTS in a ratio between 1:1 and 1:5, preferably between 1:1 and 1:3, more preferably between 1:1 and 1:2.

8. The method of any one of claims 1 or 7, wherein the amplifier is a dsDNA lacking a PAM motif, comprising the target sequence of a Cas12 A-RNP, and wherein the NTS has a 3' extension of ssDNA and/or RNA terminally protected from exonucleolytic degradation; and wherein an exonuclease is added in step (a), optionally wherein a phosphatase is also added in step (a).

9. The method of any one of claims 1 to 8, wherein the reporter is a nucleic acid molecule with a fluorescent marker in one end and a quencher in the other end.

10. The method of any one of claims 1 to 9, wherein step (a) is performed in substeps:
(a1) adding to said sample at least one T-RNP with collateral activity and at least one amplifier,
(b1) mixing and incubating the mixture of step (a1) between 1 minute and 2 hours, preferably between 10 minutes and 1 hours, more preferably between 20 minutes and 40 minutes, at a temperature between 15 to 45 ºC, preferably between 20 to 40 ºC, more preferably between 25 and 37 ºC,
(a2) optionally, adding more amplifier and/or at least one of its components (TS and NTS), preferably adding additional NTS of the amplifier,
(b2) optionally, mixing and incubating the mixture of step (a2) between 1 minute and 1 hour, preferably between 10 minutes and 1 hour, more preferably between 15 minutes and 30 minutes, at a temperature between 15 to 45 ºC, preferably between 20 to 40 ºC, more preferably between 25 and 37 ºC,,
(a3) adding at least one A-RNP, at least one reporter substrate, and at least one of an exonuclease or a polymerase, optionally combined with dNTPs and/or NTPs, and
(b3) incubating the mixture of step (a3) between 1 minute and 8 hours, preferably between 15 minutes and 2 hours, more preferably between 30 minutes and 1.5 hours, at a temperature between 15 to 45 ºC, preferably between 20 to 40 ºC, more preferably between 25 and 37 ºC.

11. The method of any one of claims 1 or 7 or 9 or 10, wherein the T-RNP comprises Lwa Cas13a protein and guide RNA of SEQ ID NO: 6, the A-RNP comprises Lba Cas12a protein and guide RNA of SEQ ID NO: 3, and the amplifier comprises SEQ ID NO:4 and SEQ ID NO: 5 as TS and NTS, respectively.

12. A kit for the detection of the presence or absence or for the quantification of a nucleic acid of interest in a sample, comprising at least one Cas RNP with collateral activity, at least one amplifier, at least one reporter substrate and, optionally, at least one of an exonuclease or a polymerase, optionally combined with dNTPs and/or NTPs,
wherein at least one Cas RNP (target-activatable RNP; T-RNP) is designed to recognize a specific sequence in the nucleic acid of interest (primary target);
wherein the amplifier is a nucleic acid comprising:
i) a non-target strand (NTS) that comprises a sequence that is reverse-complementary to the target of a second Cas RNP (amplifier-activatable RNP; A-RNP); and
ii) a target strand (TS) that comprises a sequence that is, at least in part, reverse-complementary to the NTS;
wherein the 5', 3' or both ends of at least one strand of the amplifier are modified by chemical modification or by circularization;
wherein the reporter substrate comprises the type of nucleic acid that is target for the collateral activity of the A-RNP, optionally wherein its ends are protected against degradation or polymerization; and
wherein the kit does not comprise means for amplifying the nucleic acid sequence of interest in the sample.

13. The kit of claim 12, wherein the kit comprises a lateral-flow device comprising a solid support and wherein the reporter is a nucleic acid molecule with two different marker molecules at each end, preferably biotin and fluorescein, and wherein the kit optionally comprises means for taking a sample and/or for extracting nucleic acids from a sample.

14. Use of the method of claims 1 to 11 or of the kit of claims 12 or 13, for the detection of the presence or absence or for the quantification of a nucleic acid of interest in a sample, preferably wherein said nucleic acid is present in the sample in a concentration of between 100 fM and 10 nM.

15. Use of the method of claims 1 to 11 or of the kit of claims 12 or 13, for the diagnosis or prognosis of a disease or condition, wherein the presence or absence or amount of the nucleic acid is associated to said disease or condition.
